# DEMANDE DE BREVET EUROPEEN

(11) **EP 0 621 048 A1**
(43) Date de publication de la demande: **26.10.1994**
(21) Numéro de dépôt: 94490018.2
(22) Date de dépôt: 22.04.1994
(51) Int. Cl.: A61M 5/32, B65F 1/16

(54) **Boite pour objets médicaux contaminés**

(30) Priorité: 22.04.1993 FR 9304764
(71) Demandeur: DISTRI CLUB MEDICAL, Société Anonyme:, F-68990 Heimsbrunn (Haut-Rhin) (FR)
(72) Inventeur: Moutier, Antoine, F-76210 Bolbec, Seine-Maritime (FR)
(74) Mandataire: Duthoit, Michel Georges André

(57) **Abrégé**

Boîte pour petits objets, notamment objets médicaux pouvant être contaminés tels que des aiguilles (81) ou des seringues (82), constituée d'un boîtier (20) et d'un couvercle (10) lesquels comportent chacun sur sa face supérieure une ouverture partielle (11, 21), le couvercle (10) étant monté rotatif sur le boîtier (20) sans changer de niveau pour, à la demande, passer alternativement d'une position ouverte à une position fermée en disposant, ou non, en vis-à-vis les deux ouvertures (11, 21), et telle qu'une ouverture partielle (11) est munie d'un puits protecteur (12) à paroi sensiblement verticale et d'une languette (13) avec fente (14) pour faciliter un dégoupillage protégé de l'aiguille (81) de la seringue (82).

## Description

La présente invention a pour objet une boîte pour petits objets, notamment objets médicaux pouvant être contaminés tels que des aiguilles ou des seringues, constituée d'un boîtier et d'un couvercle lesquels comportent chacun sur sa face supérieure une ouverture partielle, le couvercle étant monté rotatif sur le boîtier sans changer de niveau pour, à la demande, passer alternativement d'une position ouverte à une position fermée en disposant, ou non, en vis-à-vis les deux ouvertures partielles.

Une telle boîte trouve son utilisation dans les milieux médicaux où l'on manipule souvent des objets tels que des seringues avec des aiguilles qui, après utilisation, peuvent se trouver contaminés; il convient donc de réduire au minimum leur manipulation après usage et bien sûr de ne pas les jeter négligemment dans une poubelle mais au contraire de les mettre en lieu sûr par exemple dans une boîte que l'on peut ouvrir puis refermer après y avoir mis l'objet potentiellement dangereux. Dans le cas des seringues avec une aiguille, la plupart du tremps seule l'aiguille est contaminée et on cherche à récupérer la seringue pour un usage ultérieur en dégoupillant l'aiguille; cette opération de dégoupillage est particulièrement dangereuse malheureusement elle s'effectue encore souvent en prenant l'aiguille dans une main et la seringue dans l'autre pour les séparer.

La présente invention a pour but de procurer une boîte comportant un dispositif qui permet de dégoupiller une aiguille sans jamais avoir besoin de la prendre à la main ; la présente invention a aussi pour but de procurer une boîte qui après plusieurs utilisations, notamment lorsqu'elle est pleine, comporte un dispositif supplémentaire de fermeture agencé pour empêcher toute ouverture ultérieure de la boîte.

Selon une première caractéristique de la présente invention, une boîte pour petits objets telle que définie précédemment est particulièrement remarquable en ce qu'une ouverture partielle est munie d'un puits protecteur à paroi sensiblement verticale et d'une languette avec fente pour faciliter un dégoupillage protégé de l'aiguille de la seringue. Ainsi, il est maintenant possible d'introduire l'aiguille non dégoupillée dans le puits protecteur puis de coincer l'aiguille dans la fente de la languette et de procéder au dégoupillage en tirant sur la seringue ce qui provoque la chute de l'aiguille directement dans la boîte sans aucune manipulation dangereuse.

Selon un mode de réalisation préféré, l'ouverture partielle qui est munie d'un puits est l'ouverture partielle du couvercle de la boîte. Dans la description qui suit et dans les dessins annexés seul un puits protecteur pratiqué dans l'ouverture partielle du couvercle de la boîte est décrit, mais il est clair qu'on peut aussi envisager de disposer le puits protecteur dans l'ouverture partielle du boîtier.

Selon la présente invention, une boîte pour petits objets comporte en outre une position de fermeture définitive, laquelle est prévue et agencée pour empêcher toute ouverture ultérieure de la boîte.

Préférentiellement, à partir de la position fermée, le couvercle est amené dans la position de fermeture définitive par un changement de niveau jusqu'à atteindre un encliquetage irréversible sur le boîtier pour empêcher tout changement de niveau ultérieur. Ainsi, le couvercle se trouve dans une position qui l'empêche de revenir dans sa position initiale pour son usage habituel.

Préférentiellement, le changement de niveau s'effectue par enfoncement vertical vers le bas du couvercle sur le boîtier, enfoncement pendant lequel le puits du couvercle s'engage dans un profil borgne similaire aménagé dans le boîtier pour empêcher toute rotation ultérieure du couvercle. Ainsi la boîte une fois fermée définitivement comporte une double sécurité qui condamne définitivement toute tentative d'ouverture.

Avantageusement, on peut prévoir que la fente de la languette comporte des crans étagés pour le dégoupillage d'aiguilles de différentes tailles; de même, on peut prévoir que le mouvement de rotation du couvercle entre la position ouverte et la position fermée comporte deux encliquetages périphériques pour le maintien provisoire du couvercle dans l'une ou l'autre position; ainsi la manipulation de la boîte lors des dégoupillages successifs est facilitée; ce qui est bien sûr un gage de sécurité, puisque d'une part le couvercle n'est pas complètement libre en rotation du fait de l'encliquetage, et d'autre part on peut choisir pour le dégoupillage le cran dont la dimension correspondant à celle de l'aiguille.

La présente invention sera bien comprise au vu de la description non limitative d'un exemple de réalisation illustré par les dessins annexés parmi lesquels:
- la figure 1 représente un couvercle selon l'invention en vue de dessus associé à deux vues de face en coupe selon la ligne A-A et la ligne B-B,
- la figure 2 représente un boîtier conforme à l'invention en vue de dessus associé à une vue de face en coupe selon la ligne C-C,
- la figure 3 représente la boîte assemblée conforme à l'invention vue en coupe en position ouverte,
- la figure 4 représente similairement la boîte en position fermée,
- la figure 5 représente similairement la boîte en position de fermeture définitive,
- la figure 6 représente un détail du système d'encliquetage du couvercle sur le boîtier,
- la figure 7 illustre la position de la main en appui sur le couvercle pour une opération de dégoupillage,
- la figure 8 illustre la position d'une seringue dont l'aiguille est coincée pour son dégoupillage,

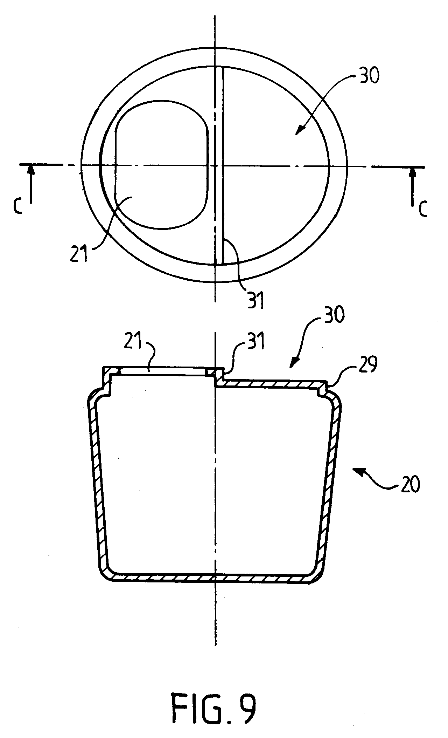

- la figure 9 représente une variante du boîtier conforme à l'invention vue de dessus associé à une vue de face en coupe.

Dans la description qui suit, d'une figure à l'autre, les mêmes repères numériques sont utilisés pour les mêmes éléments.

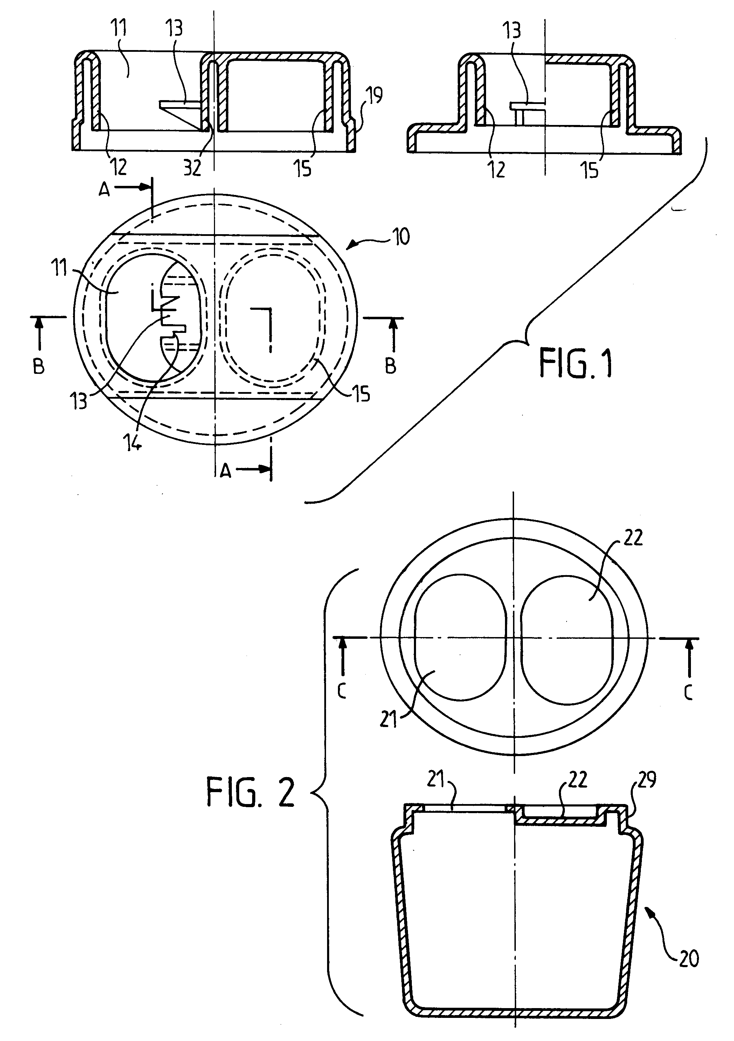

La figure 1 représente un couvercle 10 en vue de dessus; il a une forme généralement cylindrique avec en partie basse une jupe 19, et sa partie supérieure comporte une ouverture partielle 11 continuée vers le bas par un puits protecteur 12 à paroi sensiblement verticale, une partie de paroi comportant, en avancée, une languette 13 munie d'au moins une fente 14 laquelle est ici représentée avec des crans étagés, d'autres formes de fente sont évidemment envisageables. Les deux coupes selon les lignes A-A et B-B représentées en vue de profil permettent de bien voir l'agencement du couvercle 10 lequel peut clairement être moulée par exemple dans une matière plastique; de manière optionnelle, comme décrit ci-après, on peut prévoir de disposer similairement au puits protecteur 12, mais sur l'autre moitié du couvercle un autre puits 15 lequel est borgne; en cas de nécessité de moulage, on peut prévoir que cet autre puits 15 est obtenu avec une pièce rapportée à fixer sur le couvercle d'une manière connue.

Sur la figure 2, est représenté un boîtier 20 de forme générale cylindrique dont la face supérieure est fermée sauf qu'elle comporte une ouverture partielle 21 dont la forme doit évidemment être similaire à celle de l'ouverture partielle 11 du couvercle 10; dans sa partie supérieure, le boîtier 20 comporte une surface cylindrique 29 dont le diamètre extérieur correspond au diamètre intérieur de la jupe 19 et du couvercle 10; symétriquement à l'ouverture partielle 21 de la partie supérieure du boîtier 20, il est prévu un profil borgne 22 agencé en creux dans la partie fermée de la partie supérieure du boîtier 20, ce profil borgne 22 a aussi la même forme, au moins partiellement, que la partie basse du puits 12 du couvercle 10.

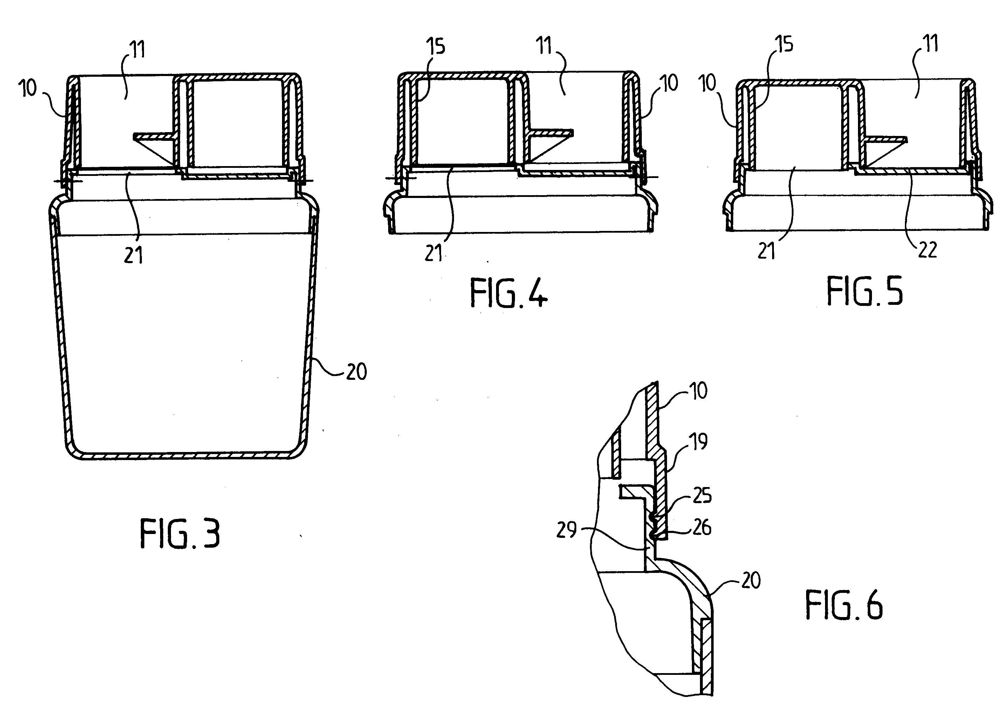

La figure 3 représente la boîte dans son ensemble en position ouverte constituée du boîtier 20 sur lequel est emmanché le couvercle 10, les ouvertures partielles 21 et 11 étant disposées en vis-à-vis. Il est clair que dans cette position qui constitue la position ouverte des objets peuvent être introduits dans la boîte.

La figure 4 représente, partiellement, la même boîte que celle de la figure 3 mais maintenant en position fermée puisque les ouvertures 11 et 21 ne sont plus en vis-à-vis du fait de la rotation du couvercle d'un demi-tour, cette rotation étant évidemment possible du fait des diamètres respectifs de la partie cylindrique 29 du boîtier 20 et de la jupe 19 du couvercle 10. Dans cette position fermée, il est clair qu'aucun objet ne peut être introduit dans la boîte mais surtout qu'aucun objet préalablement introduit dans la boîte ne peut en sortir; on voit ici l'intérêt du puits borgne 15 prévu dans le couvercle 10 pour empêcher des objets déjà introduits dans la boîte d'aller se loger dans le couvercle au cas où la boîte serait retournée.

Sur la figure 5, la même boîte est à nouveau représentée partiellement mais maintenant en position de fermeture définitive; on voit qu'à partir de la position fermée de la figure 4, le couvercle 10 a été enfoncé vers le bas de sorte que, d'une part le puits borgne 15 du couvercle 10 s'est engagé dans l'ouverture partielle 21 du boîtier 20, d'autre part le puits protecteur 11 du couvercle 10 s'est engagé dans le profil borgne 22 du boîtier 20 de sorte qu'il est maintenant impossible de faire pivoter le couvercle 10 sur le boîtier 20 sans préalablement ramener le niveau du couvercle à un niveau supérieur tel que représenté sur les figures 3 et 4 où sa rotation est libre.

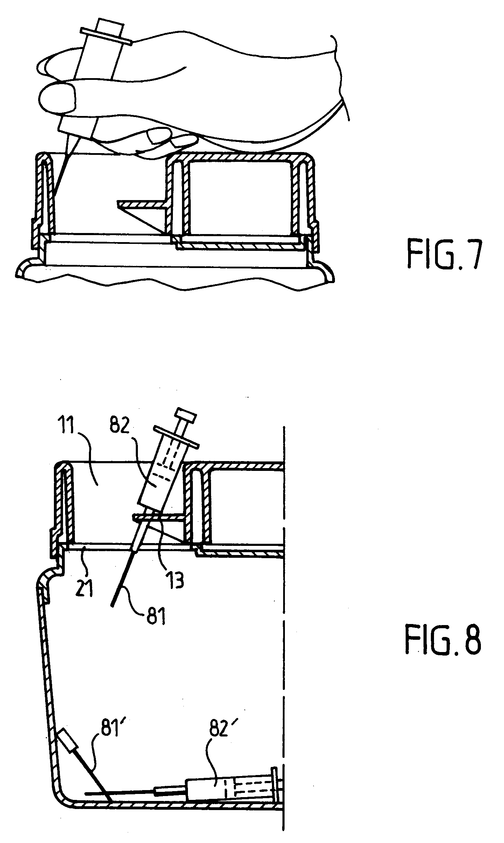

On montre à la figure 7 une variante de réalisation du profil borgne 22 qui, dans ce cas, se présente sous la forme d'un dénivellé 30. Le verrouillage en position fermé s'effectue alors par la coopération d'un méplat 31 du dénivellé 30 et d'une face 32 du puits 11.

La figure 6 est un détail agrandi de la liaison boîtier-couvercle; on y voit en effet une partie du couvercle 10 avec sa jupe 19, et aussi une partie du boîtier 20 avec sa partie cylindrique 29; des rainures et des crans 25, 26 sont aménagés respectivement dans le boîtier 20 et dans le couvercle 19 pour coopérer de manière connue et obtenir deux effets d'encliquetage et de guidage; dans la position représentée sur la figure 6, qui est celle des figures 3 et 4, le couvercle 10 est maintenu à un premier niveau par la rainure 25 aménagée dans le boîtier 20 et le couvercle 10 est libre en rotation; avantageusement on peut prévoir deux points durs correspondant chacun à la position ouverte de la figure 3 ou à la position fermée de la figure 4 pour maintenir provisoirement le couvercle dans l'une ou l'autre position; ainsi que déjà mentionné précédemment, à partir de la position fermée de la figure 4, il est prévu d'enfoncer le couvercle 10 vers le bas en profitant de l'élasticité de la jupe 10 pour amener le couvercle 10 à un niveau inférieur jusqu'à atteindre un encliquetage irréversible dans la rainure 26 dont le profil est aménagé en conséquence de manière connue. Ainsi, le couvercle 10 ne peut plus revenir au niveau supérieur et, du fait qu'il ne peut plus non plus tourner à cause des engagements déjà mentionnés 15 dans 21 et 11 dans 22, la boîte se trouve scellée dans une position de fermeture définitive.

Afin d'éviter l'encliquetage du couvercle sur le boîtier en position ouverture, on peut prévoir un ou plusieurs détrompeurs notamment au niveau de la partie cylindrique 29 pour n'autoriser l'enfoncement que lorsque le puits 11 est en face du profil borgne 22.

On peut aussi noter sur la figure 6 que le boîtier 20 peut être fabriqué en deux parties, telles que visibles sur la figure, chaque partie pouvant être moulée séparément puis assemblée de manière connue.

Les figures 7 et 8 illustrent l'utilisation de la boîte conforme à l'invention; la face supérieure du couvercle sert d'appui pour la paume de la main qui tient la seringue sans avoir besoin de toucher l'aiguille; l'aiguille 81 est introduite dans le puits protecteur 11 et sa base est engagée dans une fente de la languette 13, laquelle est à une distance adéquate de la face supérieure du couvercle, de sorte qu'une simple traction vers le haut sur la seringue 82 avec appui de la main pour faire bras de levier, permet de dégoupiller l'aiguille laquelle tombe directement. Si le dégoupillage n'est pas nécessaire, ou impossible, on peut introduire toute la seringue 82 dans le boîtier.

Au vu de la description qui précède, il est clair qu'on peut envisager des variantes sans toutefois sortir du cadre de la présente invention; par exemple on peut prévoir que le puits protecteur 12 est aménagé à partir de l'ouverture partielle 21 de la boîte 20 et non pas dans le couvercle comme décrit; on peut aussi prévoir un seul des deux engagements anti-rotation mentionnés c'est-à-dire soit l'engagement 15 dans 21 soit l'engagement 11 dans 22.

Par ailleurs, afin de pouvoir conforter la stabilité du boitier, notamment pendant la phase de dégoupillage des aiguilles, le boîtier 20 peut avantageusement porter à sa partie inférieure des ventouses coopérant avec la surface de pose.

La force d'adhésion et le nombre de ces ventouses sont adaptés en fonction de l'état de la surface sur laquelle est posée ladite boîte.

## Revendications

1. Boîte pour petits objets, notamment objets médicaux pouvant être contaminés tels que des aiguilles ou des seringues, constituée d'un boîtier (20) et d'un couvercle (10) lesquels comportent chacun sur sa face supérieure une ouverture partielle (11, 21), le couvercle étant monté rotatif sur le boîtier sans changer de niveau pour, à la demande, passer alternativement d'une position ouverte à une position fermée en disposant, ou non, en vis-à-vis les deux ouvertures partielles (11, 21), caractérisée en ce qu'une ouverture partielle est munie d'un puits protecteur (12) à paroi sensiblement verticale et d'une languette (13) avec fente pour faciliter un dégoupillage protégé de l'aiguille de la seringue.

2. Boîte pour petits objets selon la revendication 1, caractérisée en ce que l'ouverture partielle munie d'un puits (12) est l'ouverture partielle (11) du couvercle de la boîte.

3. Boîte pour petits objets selon la revendication 1 ou 2, caractérisée en ce qu'elle comporte en outre une position de fermeture définitive, laquelle est prévue et agencée pour empêcher toute ouverture ultérieure de la boîte.

4. Boîte pour petits objets selon la revendication 3, caractérisée en ce que, à partir de la position fermée, le couvercle est amené dans la position de fermeture définitive par un changement de niveau jusqu'à atteindre un encliquetage irréversible (26) sur le boîtier pour empêcher tout changement de niveau ultérieur.

5. Boîte pour petits objets selon la revendication 4, caractérisée en ce que le changement de niveau s'effectue par enfoncement vertical vers le bas du couvercle sur le boîtier, enfoncement pendant lequel le puits du couvercle (12) s'engage dans un profil borgne (22) aménagé dans le boîtier pour empêcher toute rotation ultérieure du couvercle.

6. Boîte pour petits objets selon l'une quelconque des revendications précédentes, caractérisée en ce que la fente (14) de la languette (13) comporte des crans étagés pour le dégoupillage d'aiguilles de différentes tailles.

7. Boîte pour petits objets selon l'une quelconque des revendications précédentes, caractérisée en ce que le mouvement de rotation du couvercle entre la position ouverte et la position fermée comporte deux encliquetages périphériques (28) pour le maintien provisoire du couvercle dans l'une ou l'autre position.

8. Boîte pour petits objets selon l'une quelconque des revendications précédentes, caractérisée en ce que la languette (13) est à une distance choisie de la face supérieure du couvercle de sorte que le dégoupillage de l'aiguille s'effectue avec un seul geste de la main.
